# EUROPEAN PATENT SPECIFICATION

(11) **EP 0 386 048 B1**
(45) Date of publication and mention of the grant of the patent: **21.09.1994**
(21) Application number: 88909333.2
(22) Date of filing: 21.10.1988
(51) Int. Cl.: A61M 1/30, A61M 1/34

(54) **BLOOD PURIFICATION APPARATUS**
BLUTREINIGUNGSVORRICHTUNG
APPPAREIL D'EPURATION DU SANG

(30) Priority: 23.10.1987 GB 8724914
(43) Date of publication of application: 12.09.1990
(73) Proprietor: 3i RESEARCH EXPLOITATION LIMITED, London SE1 8XP (GB)
(72) Inventor: GREENWOOD, Roger, Newton, London NW6 3EU (GB); ALDRIDGE, Colin, London E4 7PB (GB); TATTERSALL, James, Erskine, London SW17 8DF (GB); BARRETT, Rodney, Valentine, Bristol BS6 6PZ (GB)
(74) Representative: March, Gary Clifford
(86) International application number: PCT/GB88/00920
(87) International publication number: WO 89/03696

(56) References cited:
- EP-A- 0 105 845
- EP-A- 0 114 698
- EP-A- 0 132 210
- US-A- 4 331 540
- A. S. A. I. O. Transactions, volume 33, no. 3, September 1987, ( Hagerstown, MD, US), R. R. Stromberg et al.: "Development of a novel membrane apheresis system for plasma collection at mobile sites", pages 614 - 620 see page 614, column 2, lines 8 - 28; page 615, figure 1; page 617, column 1, line 28 - coulmn 2, line 12; page 618, figure 5, column 1, lines 1 - 9

## Description

This invention relates to apparatus for the purification of blood, for example, by extracorporeal haemodialysis and/or haemofiltration and/or haemoperfusion. Haemodialysis is a widely used medical treatment for patients with acute and chronic renal failure. Haemofiltration is an alternative treatment gaining in popularity which, in its intermittent form, involves massive removal of plasma water (approximately 30 litres) and replacement with sterile electrolyte solution. Haemoperfusion is a process for detoxifying blood in,for example, drug overdose, by contacting the blood with an adsorbent, such as activated carbon, to remove the toxins.

Haemodialysis services are normally supplied on an out-patient basis in specially constructed renal dialysis units or carried out at home by the patients themselves. Patients typically require three sessions of 4-6 hour duration per week. The hardware associated with haemodialysis usually consists of a fixed installation requiring fluid supplies, drainage and control apparatus.

BE-A-905.407 describes a single-needle haemodialysis system in which blood withdrawn by a pump from a patient suffering from renal insufficiency is passed through a dialyser into an expansion chamber. The expansion chamber is in the form of a solid-walled, pressure resistant vessel having a line through which blood is delivered to and withdrawn from the vessel and also an air line extending from the vessel to a pressure sensor which has an associated control system operatively connected to the pump. As blood is delivered into the chamber by the pump the pressure of air, trapped in the vessel above the blood level, increases and is sensed by the sensor. When a preselected pressure is sensed by the pressure sensor, the control system sends a signal to the pump to reverse the direction of pumping thereby withdrawing the blood from the expansion chamber and returning it, via the dialyser and pump, to the patient. Thereafter, when the sensed pressure falls to a preselected lower limit the control again reverses the direction of pumping to complete the cycle. A disadvantage of the proposed system is potential dancer from control failure whereby dangerous, even fatal, volumes of air might be introduced into the patient's circulation.

EP-A-105,845 discloses a blood purification system in which blood is withdrawn from and returned to a patient through a dialyser. A pneumatically operated diaphragm withdraws blood through pressure reduction and returns blood under pressure. Precise pneumatic control of blood flow can be difficult and the blood chamber requires a rigid outer construction.

### EP 0 132 210 (RHONE-POULENC)

Apparatus for plasmapheresis of blood is described wherein treated blood is collected within a flexible reservoir 17, pressure being applied thereto by an inflatable bag 18 to maintain a positive pressure and to prevent reverse filtration of treatment fluid from bag 20 or air from duct 30 into the blood compartment. A peristaltic pump 7 is used to transport blood in both directions into and out of the treatment module 3. The inflatable bag 18 is expanded by means of compressor 23 which does not control blood flow into and out of the treatment module 3, this being the task of the peristaltic pump 7.

### A.S.A.I.O. TRANSACTIONS - CITED IN THE INTERNATIONAL SEARCH REPORT

Discloses an apparatus for plasma collection wherein a flexible blood reservoir is compressed by gaseous means to direct blood one way through a purification module; gravity is used to control flow in the other phase. This document also fails to disclose an apparatus wherein hydraulic fluid is used to act on the blood reservoir to control flow into and out of the purification module.

The most widely used haemodialysis system involves withdrawal of blood from the patient via a needle introduced into a vein supplied by a surgically created arteriovenous fistula, establishing a flow, using a peristaltic pump through a dialyser with return to the patient's circulation via a second needle. Alternatively, a single needle is used in which case blood is withdrawn from and returned to the fistula alternately by using twin pumps. A flow of dialysis fluid is established through the dialyser across that side of the membrane remote from the blood and thence to drain. To achieve sufficient dialysis within acceptable time periods, flow rates of both blood and dialysis fluid are high, typically from 200 ml/min to 400 ml/min for blood and 500 ml/min for dialysis fluid. The extracorporeal blood volume of the dialysis system is high (200-450 ml) and large volumes of the dialysis fluid are required (120-160 litres per dialysis). The overall size of the installation and the complexity of the instrumention is consequently great. Such requirements with the need for large volumes of dialysis fluid are in conflict with a need to produce portable dialysis apparatus.

The foregoing description applies particularly to treatment of chronic renal failure. However, in acute renal failure being a reversible condition occasionally following trauma, surgery or severe illness, patients may require blood purification during their stay in intensive care units. In this setting, bulky dialysis machines with associated parts and special plumbing are inconvenient because of competition for space with other intensive care equipment such as respirators, drips and vital signs monitors. Also the complexities of operation of traditional dialysis machines cause problems for intensive care nursing staff who may not have been trained in renal units.

An object of the present invention is to obviate or mitigate the aforesaid disadvantages.

According to the present invention there is provided blood purification apparatus in accordance with claim 1.

The apparatus most preferably is a closed or dead-end system.

In use, upon connecting the apparatus to a patient, blood can flow under its own pressure to at least partially fill the flexible reservoir, without any external pressure or assistance, e.g. from the pump assembly if present. Conveniently the flexible reservoir consists of a conventional blood bag as used in transfusion.

Preferably the blood purification module comprises one or more of a dialyser, haemofilter, haemoperfusion unit, an oxygenator and/or plasma filter.

Preferably, the blood purification module is, or includes, a dialyser and said apparatus includes means for passing dialysis fluid through the dialyser. In a preferred embodiment, these means comprise a first reservoir for fresh dialysis fluid, a pump for passing fluid from the first reservoir to the dialyser and a second reservoir for receiving and holding the entire effluent dialysis fluid from the dialyser. It is preferred that the dialyser be of the hollow fibre membrane type.

In a preferred embodiment of the invention, the means for controlling blood flow to and from the patient comprises a piston pump assembly. This may include a first chamber for hydraulic fluid, a second chamber separated from the first by a common flexible wall member, within the second chamber a flexible blood bag disposed proximate the flexible wall and in fluid flow communication with the blood purification module, and piston means within the first chamber disposed to act upon the hydraulic fluid. The piston is preferably motor driven, advantageously by a microprocessor controlled stepper motor.

The apparatus of the invention may also include means for injecting physiological fluid, such as an electrolyte solution, into the purified blood before return to the patient.

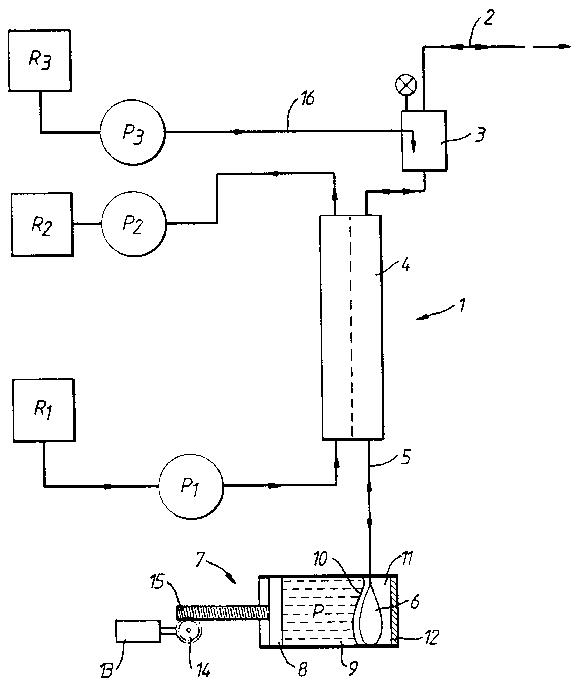

The invention will now be described, by way of example, only with reference to the accompanying drawing which shows a flow diagram of an embodiment of apparatus according to the invention.

Blood purification apparatus of the invention 1 has a blood conduit 2 for connection to a patient's blood supply via an intravenous needle or catheter (not shown). The conduit 2 transports blood from the patient to a bubble trap 3 and thereafter to one side of the membrane (dotted lines) of a dialyser, haemofilter or other purification module 4. Blood line 5 supplies blood from the dialyser module 4 to a blood bag 6 in reversible flow.

Blood bag 6 is located within a pump assembly P, indicated generally as 7, which includes a piston 8 to act hydraulic fluid in a first pump chamber 9 to distend or relax a flexible wall member 10. A second chamber 11 is formed by flexible wall member 10, in common with the first chamber, and rigid end closure plate 12. The blood bag is disposed in the second chamber between the plate 12 and the flexible wall member 10 so that when the piston 8 acts upon the hydraulic fluid in the first chamber 9, the flexible wall member 10 distends and exerts a sufficient squeezing action on the bag 6 to overcome the resistance of the blood circuit, to return the blood to the patient. As piston 8 retracts blood flows into the conduit 2, 5 and into the bag 6 as a result of the negative pressure created by pump P in chamber 9.

The piston 8 may be motor driven by a motor 13 driving a worm gear 14 against a helically threaded piston shaft 15. It is convenient to use a stepper motor to facilitate microprocessor control of the speed and distance of travel of the piston and to monitor blood flow rates and volumes.

The apparatus 1 also includes reservoirs R₁ and R₂ dialysis fluid and pumps P₁ and P₂ for passing fluid from R₁ to R₂, via the dialyser module 4 on that side of the membrane remote from the blood. The receiving reservoir R₂ is of sufficient capacity to receive and hold the entire volume of dialysis fluid delivered from R₁ plus any additional water acquired in the dialyser by ultra-filtration from the blood. This arrangement permits accurate measurement of fluid balance across the patient.

In use, blood, is allowed to flow under the patients own pressure into the blood bag 6, flowing through the dialyser module 4 wherein dialysis against dialysis fluid flowing across the other side of the dialysis membrane occurs. Once a volume of blood has accumulated in the bag 6, then immediately or after an interval, the motor 13 is actuated for forward travel of the piston 8 to act via the hydraulic fluid in chamber 9 to displace and/or distend the flexible wall 10 to press upon the bag 6 and reverse the direction of blood flow. Thus, the blood passes through the dialyser once in a forward direction and again in the reverse direction. It is believed that intervals between forward and reverse cycles during which the blood is held stationary in the bag 6, may be advantageous to permit diffusion of solutes from the red blood cells through the cell wall into the plasma during this stationary period thus increasing the degree of dialysis during the reverse pass through the dialyser.

There are definite end points in the purification cycle, particularly at the close of the venous phase, so that extracorporeal blood cannot be continuously extracted which has been a risk with peristaltic pumped systems. The proximal and distal dead spaces can also be reduced to a minimum.

In one intended application, e.g. during intensive care, the degree of treatment achieved on each pass need not be high as the treatment may be carried out continuously. Flow rates of blood and dialysis fluid may be comparatively low and the volume of dialysis fluid can be low. This permits very gentle blood treatment. It is known that blood damage such as haemolysis leading to trauma is a danger with extracorporeal treatments; this slow, gentle flow is a desirable feature, minimising such damage. As a consequence of the low volumes of dialysis fluid which have to be handled, prepackaged sterile fluid packs may be used and the whole apparatus made self-contained in terms of its fluid requirements. Thus the apparatus may be portable and have small space requirements, making it eminently suitable for use in the equipment-crowded environment of an intensive care unit or as a part of the equipment carried on an ambulance.

The compact uncomplicated arrangement possible with this apparatus facilitates automatic control of the purification procedure. The pump assembly 7 may be driven by a microprocessor controlled stepper motor from which the rate of treatment and volume of blood treated may be monitored. This may be determined by counting the number and rate of revolution of the drive shaft using, for example, an optical interruption technique. Should a residence time for the blood in the blood bag be desired this may easily be achieved by stopping the motor for the requisite time, under microprocessor control, before reversing its drive direction.

Using a pump at either end of the dialyser allows the direction of flow of water across the membrane to be reversed in order to rehydrate the blood. It appears that reversal of the direction of flow of water across the dialyser membrane may have a beneficial effect on the useful life of the dialyser. It is well known that during use the transport characteristics of dialysis membranes deteriorate with time because of fouling of the pores by large biomolecules such as proteins and lipids. There is strong evidence that reversal of the direction of ultrafiltration may dislodge some of the trapped fouling biomolecules thus reopening the pores and restoring the permeability of the membrane.

The invention has been described with reference to haemodialysis but it may alternatively or additionally be used in a haemoperfusion system. The dialysis module 4 may simply be removed and replaced by a carbon-packed haemoperfusion column, in which case the flow circuit for dialysis fluid is not required. Alternatively, a haemoperfusion column may be added to the dialysis circuit in serial fluid flow with the dialysis module. Similarly, as an alternative or addition to the dialyser, a blood oxygenator or plasma separator may be used. Operating conditions for such alternative or additional units are well known.

During normal dialysis procedures a degree of removal of plasma water from the blood occurs by ultrafiltration. The ultrafiltration rate is controlled by the relative speeds and timings of P₁ and P₂. In an alternative operating mode of the apparatus of the invention, the patient may have blood purification by haemofiltration. In this process plasma water is ultrafiltered from the blood under the action of P₂. Rehydration of the blood may be effected by introduction of a sterile replacement fluid from reservoir R₃ via pump P₃ and flow line 16 into the bubble trap 3 which is a convenient location for the introduction. The timing of the pump P₃ may be selected to allow pre- and/or post-dilution haemofiltration.

It will be understood that embodiments of the apparatus may include such ancillary equipment as is common in conventional dialysis machines for example, monitoring and control modules such as flow rate indicators and blood leak detectors. The apparatus may include means for introducing anticoagulant, such as heparin, into the extracorporeal blood flow. The introduction may be appropriately timed during the blood purification cycle.

In the purification cycle, blood movement can be controlled by altering the volumetric capacity of the flexible reservoir. Such blood movement between patient and reservoir is amenable to accurate volumetric, velocity and directional control by, for example, using microprocessors to regulate the direction, frequency, force and displacement of the piston in the pump assembly. Such accurate control can be through direct input, not requiring feed back from any point in the cycle. It will be appreciated that this pump P, 7 does not act directly on the blood, but indirectly through the media of the hydraulic fluid, the flexible membrane and the exterior surface of the reservoir. Within the reservoir the blood is not subjected to shear stresses with the desirable result that less haemolysis is observed than in blood purification circuits utilising peristaltic pumps.

Furthermore the instantaneous control of volume, velocity and direction of blood flow which is possible with this apparatus allows not only precise timing of the blood purification cycle, but also permits infusions and/or drug administrations at any required time during the cycle. For example incompatible infusions can be administered at different times to prevent mixing and to ensure infusion only into blood returning to the patient. These advantages are still possible when using the same injection or infusion site. This is of particular benefit in haemofiltration. Switching between post-dilution and pre-dilution haemofiltration can be effected by altering the timing of the replacement fluid injections rather than changing the injection site as is usually necessary with other blood purification systems.

## Claims

1. Blood purification apparatus comprising means (2) for withdrawing blood from a patient into a blood conduit (1,5), a blood purification module (4) in fluid flow communication with the said conduit (1,5), a blood reservoir (6) for receiving blood from the said module (4), pump means (7) for withdrawing blood or for returning blood from said reservoir (6) to the patient via the blood purification module (4), said reservoir comprising a flexible container (6) of variable volumetric capacity located within part (11) of said pump means (7) characterised in that the pump means includes a piston (8) arranged to act on hydraulic fluid in said pump means to distend or relax a flexible wall member (10) to effect withdrawal of blood from the patient or return of blood from said reservoir (6).

2. Apparatus as claimed in claim 1, wherein the total blood volume of the apparatus is equivalent to and varies with the extracorporeal blood volume.

3. Apparatus as claimed in claim 1 or 2 in which the blood purification module comprises one or more of a dialyser, a haemoperfusion unit, a haemofilter, an oxygenator and a plasma filter.

4. Apparatus as claimed in claim 3 in which one or more of the same or different purification modules are arranged in series.

5. Apparatus as claimed in any preceding claim in which the pump means comprises a piston pump assembly having first and second chambers and wherein said reservoir is located within the second chamber of said piston pump.

6. Apparatus as claimed in any preceding claim in which the blood purification module comprises a dialyser of the hollow fibre membrane type.

7. Apparatus as claimed in any preceding claim which includes a first reservoir for fresh dialysis fluid, and additional pump means adapted to pass fluid from the first reservoir to the blood purification module and a second reservoir for receiving and holding the entire effluent dialysis fluid from the blood purification module.

8. Apparatus as claimed in claim 7, in which said additional pump means comprises one pump intermediate the first reservoir and the blood purification module and another pump intermediate the blood purification module and the second reservoir.

9. Apparatus as claimed in claim 8, in which said pumps of the additional pump means are of a type permitting selection of the direction of pumping.

10. Apparatus as claimed in any preceding claim, in which the means for withdrawing blood from and returning blood to the patient is a pump assembly comprising a first chamber for hydraulic fluid, a second chamber separated from the first by a common flexible wall member, said flexible reservoir being positioned within the second chamber proximate the flexible wall and in fluid flow communication with the blood purification module, and piston means within the first chamber disposed to act upon the hydraulic fluid.

11. Apparatus as claimed in claim 10, in which the piston is motor driven.

12. Apparatus as claimed in claim 11, in which the motor is a microprocessor controlled stepper motor.

13. Apparatus as claimed in any one of claims 5 or 10 to 12, in which the piston has a helically threaded shaft which is adapted to engage a driven worm gear.

14. Apparatus as claimed in any preceding claim, including means capable of introducing physiological fluid into the blood during return of the blood to the patient.

## Patentansprüche

1. Blutreinigungsvorrichtung mit Mitteln (2) zur Blutentnahme von einem Patienten in eine Blutleitung (1,5), einem Blutreinigungsmodul (4) in Strömungsmittelverbindung mit der genannten Leitung (1,5), einem Blutspeicher (6) für die Aufnahme von Blut aus dem genannten Modul (4), einer Pumpeinrichtung (7) für die Blutentnahme oder die Blutrückführung aus dem genannten Speicher (6) über den Blutreinigungsmodul (4) zu dem Patienten, wobei der genannte Speicher einen in einem Teil (11) der genannten Pumpeinrichtung (7) angeordneten, flexiblen Behälter (6) von veränderlichem, volumetrischem Fassungsvermögen umfaßt, dadurch gekennzeichnet, daß die Pumpeinrichtung einen zur Einwirkung auf hydraulisches Strömungsmittel in der Pumpeinrichtung angeordneten Kolben (8) zwecks Aufblähung oder Entspannung eines flexiblen Wandelements (10) enthält, um Blut aus dem Patienten abzuziehen oder aus dem genannten Speicher (6) zurückzuleiten.

2. Vorrichtung nach Anspruch 1, bei der das gesamte Blutvolumen der Vorrichtung dem außerkörperlichen Blutvolumen äquivalent ist und mit diesem variiert.

3. Vorrichtung nach Anspruch 1 oder 2, bei der der Blutreinigungsmodul einen oder mehrere Dialysatoren, eine Hämoperfusionseinheit, einen Hämofilter, ein Gerät zur Sauerstoffbeladung und einen Plasmafilter umfaßt.

4. Vorrichtung nach Anspruch 3, bei der eine oder mehrere gleiche oder unterschiedliche Reinigungsmodule in Reihe geschaltet sind.

5. Vorrichtung nach einem vorhergehenden Anspruch, bei dem die Pumpeinrichtung ein Kolbenpumpenaggregat mit einer ersten und einer zweiten Kammer umfaßt und der genannte Speicher in der zweiten Kammer der genannten Kolbenpumpe angeordnet ist.

6. Vorrichtung nach einem vorhergehenden Anspruch, bei dem der Blutreinigungsmodul einen Dialysator des Hohlfasermembrantyps enthält.

7. Vorrichtung nach einem vorhergehenden Anspruch mit einem ersten Speicher für frische Dialyseflüssigkeit und einer zusätzlichen Pumpeinrichtung zur Förderung von Flüssigkeit aus dem ersten Speicher zu dem Blutreinigungsmodul und mit einem zweiten Speicher zur Aufnahme und Speicherung der gesamten, aus dem Blutreinigungsmodul abströmenden Dialyseflüssigkeit.

8. Vorrichtung nach Anspruch 7, bei der die genannte zusätzliche Pumpeinrichtung eine Pumpe zwischen dem ersten Speicher und dem Blutreinigungsmodul und eine andere Pumpe zwischen dem Blutreinigungsmodul und dem zweiten Speicher umfaßt.

9. Vorrichtung nach Anspruch 8, bei der die genannten Pumpen der zusätzlichen Pumpeinrichtung von einem Typ sind, der die Wahl der Pumprichtung erlaubt.

10. Vorrichtung nach einem vorhergehenden Anspruch, bei der die Einrichtung für die Blutentnahme aus den Patienten und die Blutrückführung zum Patienten ein Pumpenaggregat ist, das eine erste Kammer für das hydraulische Strömungsmittel, eine durch ein gemeinsames flexibles Wandelement von der ersten Kammer getrennte zweite Kammer, wobei der flexible Speicher in der zweiten Kammer nahe der flexiblen Wandung und in Strömungsmittelverbindung mit dem Blutreinigungsmodul angeordnet ist, und eine in der ersten Kammer zur Einwirkung auf das hydraulische Strömungsmittel angeordnete Kolbeneinrichtung umfaßt.

11. Vorrichtung nach Anspruch 10, bei der der Kolben durch einen Motor angetrieben ist.

12. Vorrichtung nach Anspruch 11, bei der der Motor ein durch Mikroprozessor gesteuerter Schrittmotor ist.

13. Vorrichtung nach einem der Ansprüche 5 oder 10 bis 12, bei der der Kolben eine mit Schraubengewinde versehene Stange hat, die für den Eingriff in ein angetriebenes Schneckengetriebe eingerichtet ist.

14. Vorrichtung nach einem vorhergehenden Anspruch mit Einrichtungen zur Einführung physiologischer Flüssigkeit in das Blut während des Blutrücklaufs zum Patienten.

## Revendications

1. Appareil pour purifier le sang qui comprend des moyens (2) pour soutirer une certaine quantité de sang d'un vaisseau sanguin (1, 5) d'un patient, un module de purification qui communique directement avec ledit conduit (1, 5), un réservoir (6) pour recevoir le sang directement par ledit conduit (1, 5), un réservoir (6) pour recevoir ledit sang dudit module (4), une pompe (7) pour retirer le sang ou pour le renvoyer dudit réservoir (6) vers le patient via le module de purification (4), ledit réservoir comprenant un conteneur flexible (6), dont la capacité est variable, logé dans une partie (11) de ladite pompe (7), caractérisé en ce que ladite pompe (7) comprend un piston (8) conçu pour agir sur le fluide hydraulique qu'elle contient afin de dis tendre ou de détendre un élément de paroi flexible (10) afin de soutirer du sang dudit patient ou pour le renvoyer dans ledit réservoir (6).

2. Appareil selon la revendication 1, caractérisé en ce que le volume sanguin total de l'appareil est équivalent au volume sanguin extra-corporel et varie avec celui-ci.

3. Appareil selon la revendication 1 ou 2, caractérisé en ce que le module de purification du sang comprend un ou plusieurs dialyseurs, une unité d'hémoperfusion, un hémofiltre, un oxygénateur et un filtre de plasma.

4. Appareil selon la revendication 3, caractérisé en ce que un ou plusieurs modules de purification similaires ou différents sont montés en série.

5. Appareil selon l'une quelconque des revendications précédentes, caractérisé en ce que la pompe comprend un ensemble de pompage possédant une première et une seconde chambre et en ce que ledit réservoir est placé dans la seconde chambre de ladite pompe.

6. Appareil selon l'une quelconque des revendications précédentes, caractérisé en ce que le module d'hémopurification comporte un dialyseur comprenant une membrane à fibres creuses.

7. Appareil selon l'une quelconque des revendications précédentes, caractérisé en ce qu'il possède un premier réservoir pour du liquide de dialyse frais et une pompe auxiliaire adaptée à faire passer ledit liquide du premier réservoir dans le module d'hémopurification et un second réservoir pour recevoir et conserver tout l'effluent de dialyse provenant du module d'hémopurification.

8. Appareil selon la revendication 7, caractérisé en ce que lesdits moyens de pompage additionnels comprennent une pompe entre le premier réservoir et le module d'hémopurification et une autre pompe entre le module d'hémopurification et le second réservoir.

9. Appareil selon la revendication 8, caractérisé en ce que les pompes auxiliaires sont conçues pour permettre de choisir la direction de pompage.

10. Appareil selon l'une quelconque des revendications précédentes, caractérisé en ce que les moyens pour prélever du sang du patient et pour le réinjecter à celui-ci comprennent un dispositif de pompage comportant une première chambre pour un fluide hydraulique, une seconde chambre séparée de la première par une cloison flexible commune, ledit réservoir flexible étant placé dans ladite seconde chambre, à proximité de la paroi flexible et en communication hydraulique avec le module d'hémopurification et un piston logé dans la première chambre de façon à agir sur le fluide hydraulique.

11. Appareil selon la revendication 10, caractérisé en ce le pis ton est mû par un moteur.

12. Appareil selon la revendication 11, caractérisé en ce que ledit moteur est un moteur pas-à-pas commandé par un microprocesseur.

13. Appareil selon l'une quelconque des revendications précédentes 5 ou 10 à 12, caractérisé en ce que le piston comporte un arbre qui présente un filetage hélicoïdal adapté à engrener avec une roue de vis sans fin entraînée.

14. Appareil selon l'une quelconque des revendications précédentes, caractérisé en ce qu'il possède des moyens permettant d'introduire ou d'injecter un fluide physiologique dans le sang d'un patient au cours de la réinjection du sang de celui-ci.
